# EUROPEAN PATENT SPECIFICATION

(11) **EP 1 260 595 B1**
(45) Date of publication and mention of the grant of the patent: **13.09.2006**
(21) Application number: 02012236.2
(22) Date of filing: 05.07.1996
(51) Int. Cl.: C12Q 1/68, G01N 33/543, C12N 15/10, B01J 20/00, B01D 15/00, B03C 1/00

(54) **Nucleic acid-bondable magnetic carrier and method for isolating nucleic acid using the same**
Nukleinsäuren bindender magnetischer Träger und Verfahren für die Nukleinsäureisolierung unter dessen Verwendung
Support magnétique pour acides nucléiques et procédé pour leur isolation en utilisant le dit support

(30) Priority: 07.07.1995 JP 17248195
(43) Date of publication of application: 27.11.2002
(62) Divisional of application: 96110902.2
(73) Proprietor: Toyo Boseki Kabushiki Kaisha, Osaka-shi Osaka 530 (JP)
(72) Inventor: Uematsu, Hiroaki, c/o Toyo Boseki Kabushiki Kaisha, Ohtsu-shi, Shiga-ken (JP); Daimon, Katsuya, c/o Toyo Boseki Kabushiki Kaisha, Ohtsu-shi, Shiga-ken (JP); Yoshiga, Satoko, c/o Toyo Boseki Kabushiki Kaisha, Ohtsu-shi, Shiga-ken (JP)
(74) Representative: Vossius & Partner

(56) References cited:
- EP-A- 0 125 995
- EP-A- 0 343 934
- EP-A- 0 344 270
- EP-A- 0 389 063
- WO-A-90/06045
- WO-A-92/08133
- WO-A-93/10162
- WO-A-94/11103
- WO-A-95/04140
- WO-A-95/06652
- ALDERTON R P ET AL: "MAGNETIC BEAD PURIFICATION OF M13 DNA SEQUENCING TEMPLATES" ANALYTICAL BIOCHEMISTRY, vol. 201, no. 1, 14 February 1992 (1992-02-14), pages 166-169, XP000249684

### SEQUENCE LISTING

(2) INFORMATION FOR SEQ ID NO: 1:
   (i) SEQUENCE CHARACTERISTICS:
      (A) LENGTH: 20 base pairs
      (B) TYPE: nucleic acid
      (C) STRANDEDNESS: both
      (D) TOPOLOGY: linear
   (ii) MOLECULE TYPE: other nucleic acid, synthetic DNA
   (ix) FEATURE:
      (B) LOCATION: 1..20
      (C) IDENTIFICATION METHOD: by similarity with known sequence or to an established consensus
      (D) OTHER INFORMATION: comprising of the sequence complementary to staphylococcus sequence
   (xi) SEQUENCE DESCRIPTION: SEQ ID NO: 1:
      GAACCTCTGC TCAACAAGTT 20
(2) INFORMATION FOR SEQ ID NO: 2:
   (i) SEQUENCE CHARACTERISTICS:
      (A) LENGTH: 20 base pairs
      (B) TYPE: nucleic acid
      (C) STRANDEDNESS: both
      (D) TOPOLOGY: linear
   (ii) MOLECULE TYPE: other nucleic acid, synthetic DNA
   (ix) FEATURE:
      (B) LOCATION: 1..20
      (C) IDENTIFICATION METHOD: by similarity with known sequence or to an established consensus
      (D) OTHER INFORMATION: comprising of the sequence complementary to staphylococcus sequence
   (xi) SEQUENCE DESCRIPTION: SEQ ID NO: 2:
      AAATTTGAAA AAGGCATGAA 20
(2) INFORMATION FOR SEQ ID NO: 3:
   (i) SEQUENCE CHARACTERISTICS:
      (A) LENGTH: 27 base pairs
      (B) TYPE: nucleic acid
      (C) STRANDEDNESS: both
      (D) TOPOLOGY: linear
   (ii) MOLECULE TYPE: other nucleic acid, synthetic DNA
   (ix) FEATURE:
      (B) LOCATION: 1..27
      (C) IDENTIFICATION METHOD: by similarity with known sequence or to an established consensus
      (D) OTHER INFORMATION: comprising of the sequence complementary to staphylococcus sequence
   (xi) SEQUENCE DESCRIPTION: SEQ ID NO: 3:
      TAGAATCATC AGATAACATT TTCTTTG 27
(2) INFORMATION FOR SEQ ID NO: 4:
   (i) SEQUENCE CHARACTERISTICS:
      (A) LENGTH: 25 base pairs
      (B) TYPE: nucleic acid
      (C) STRANDEDNESS: both
      (D) TOPOLOGY: linear
   (ii) MOLECULE TYPE: other nucleic acid, synthetic DNA
   (ix) FEATURE:
      (B) LOCATION: 1..25
      (C) IDENTIFICATION METHOD: by similarity with known sequence or to an established consensus
      (D) OTHER INFORMATION: comprising of the sequence complementary to staphylococcus sequence
   (xi) SEQUENCE DESCRIPTION: SEQ ID NO: 4:
      TAGAGTAGCA CTCGAATTAG GCAGT 25

## Description

The present invention relates to a nucleic acid-bondable magnetic carrier containing magnetic- responsive particles, utilized for extracting or purifying a nucleic acid from a biological material containing the nucleic acid, or for purifying an amplified product of a nucleic acid. The present invention also relates to a method for isolating a nucleic acid from a nucleic acid-containing biological material utilizing the magnetic carrier and a magnetic field, and a kit for utilizing the method.

In conventional methods for isolating a nucleic acid using a nucleic acid-bondable magnetic carrier, it is known to utilize magnetic-responsive particles having a superparamagnetic iron oxide core covered with a polymeric silane layer to which a biocompatible molecule (for example, a nucleic acid) is covalently bonded (JP-A-60-1564).

WO-A-96/41811 refers to magnetic particles having a substantially pore-free outer glass surface, or an outer glass surface with pores having less than 10 nm diameter, and their use to isolate biological material from samples.

DE-A-43 07 262 describes a process for the preparation of magnetic polymer silica compounds in a certain form by incorporating magnetic materials in their matrix.

EP-A-0 125 995 discloses a magnetically-responsive particle comprising a magnetic metal oxide core generally surrounded by a silane coat to which molecules can be covalently coupled, a mass of the particles being dispersable in aqueous media to form an aqueous dispersion having certain properties.

WO-A-93/10162 addresses magnetic porous inorganic siliceous materials having a particle size of about 1 to about 200 µm, stated to be useful as solid supports in various chromatography, immunoassays, synthesis and other separation and purification procedures.

A method for determining a ligate concentration including the following steps is also known: (1) using magnetic- responsive particles containing superparamagnetic iron oxide covered with a polymeric silane layer to which a biocompatible molecule is capable of being bonded; (2) reacting a sample solution containing a ligate, a known amount of a labeled ligate, and the magnetic-responsive particles to which a ligate-specific ligand is bonded, so as to form a ligand-ligate complex on the magnetic-responsive particles; (3) magnetically separating the magnetic- responsive particles from the reaction solution; (4) measuring the labeled ligate which is bonded to the magnetic- responsive particles or free labeled ligate in the reaction solution; and (5) applying the measurement of the label ligate to the standard curve so as to obtain the ligate concentration. This method is described in JP-B-7-6986.

In the above-mentioned methods, in order to bond the nucleic acid to the magnetic- responsive particles, it is necessary to form a silane layer to which a biocompatible molecule (for example, a nucleic acid) is covalently bonded.

Furthermore, an analyzing method and apparatus are known utilizing a sequence of a nucleic acid which is bonded to a material sensitive to the magnetic field (WO-A-86/05815). The method utilizes a magnetic or magnetizable particle covered with a material which is capable of bonding to a single strand nucleic acid so as to separate and detect the single strand nucleic acid. More specifically, the surface of the magnetic particle is covered with nitrocellulose, which is a type of cellulose derivative, and nitrocellulose is specifically bonded to a single strand DNA or RNA. The single strand DNA or RNA collected by the method is utilized for sequencing.

In the method, it is necessary to specifically bond the single strand DNA or RNA to the magnetic carrier.

It is also known to utilize a poly-cationic substrate for purifying, separating and hybridizing a nucleic acid, especially for purifying and separating a nucleic acid containing contaminants (JP-A-1-502319). In the method, a sample solution containing contaminants is contacted with the poly-cationic solid support (magnetic-responsible particle) so as to non-covalently bond the nucleic acid to the support without excessively bonding contaminants contained in the sample solution to the support. The support to which the nucleic acid has been bonded is then separated from the solution. Examples of the support include metal oxide, glass and polyamide. Examples of the poly-cationic magnetic- responsive particles include magnetic microspheres (typically, magnetic amine microspheres). The bond between the nucleic acid and the support is considered to be based on an ionic bond between the magnetic amine microspheres having a positive charge and a sugar phosphate principal chain in the nucleic acid having a negative charge.

Furthermore, it is also known a method for isolating a substance of interest in biological material utilizing magnetic particles consisting of a polymeric inner core particle and a magnetic- responsive metal oxide/polymer coating uniformly covering the core particle (JP-A-2-501753).

The method includes the steps of reacting the magnetic particles with a biological material so as to form a complex consisting of the magnetic particles and a substance from the biological material; separating the complex from the biological material; and removing the magnetic particles from the complex so as to obtain the substance.

In the method, a polymer such as polystyrene is used as an inner core particle, and metal oxide and a polymer such as polystyrene uniformly cover the inner core particle.

Superparamagnetic particles with a plurality of separated oligonucleotide sequences having monodispersibility (less than 5% of particle diameter distribution), and a method for producing magnetic particles which covalently bond or adsorb the oligonucleotides to functional groups (for example, biotinyl groups) or molecules on the surface thereof is known. It is also known to utilize a particle to which oligonucleotide is covalently bonded or adsorbed as a probe of a nucleic acid (WO-A-90/06045). The object of the method is to specifically form covalently bonds or adsorb a probe of a nucleic acid utilized for hybridization to the particle. Therefore, the above-mentioned particles are not a carrier which non-specifically immobilizes (i.e., bonds or adsorbs) a large amount of nucleic acids.

As described above, in the methods utilizing a silane or polymeric layer on the surface of the magnetic particle carrier, a nucleic acid is, for example, covalently bonded to the silane or polymeric layer on the carrier surface. Such methods require providing functional groups on the carrier (magnetic particle) surface. Accordingly, while such methods are advantageous to the separation or the quantitation of nucleic acids utilizing the specific adsorption thereof, they are not suitable for a solid phase carrier which non-specifically adsorbs a large amount of nucleic acids so as to produce a high yield.

In the case of utilizing surface-coated magnetic particles as a solid phase carrier for isolating a nucleic acid, a large particle (for example, having a diameter of more than 20 µm) is capable of responding to a weak magnetic field or a small magnetic field variation; however, it tends to rapidly precipitate and have insufficient dispersibility. Therefore, it is difficult for such large particles to adsorb and immobilize a small nucleic acid such as plasmid DNA in the reaction which requires homogeneity such as a solid phase adsorption. Furthermore, large particles have a smaller specific surface per weight than that of small particles. As a result, a large particle is only capable of bonding a small amount of biological material thereto.

Small particles (for example, having a diameter of less than 0.1 µm) have outstanding specific surface and dispersibility; however, they have insufficient settling properties. Therefore, when small particles are used in a separation utilizing the magnetic field, a larger and more expensive magnet having a larger magnetic charge is required, and it takes a longer time to separate the particles utilizing magnetic field.

Regarding a method utilizing silica particles for purifying a nucleic acid, a column separation essentially utilizing high performance liquid chromatography (HPLC) apparatus has been conventionally used. A desired nucleic acid is adsorbed to the silica carrier surface by passing a synthesized nucleic acid or an amplified product through the column. An impurity can be washed away by rinsing with a washing buffer. The desired nucleic acid can be collected using a buffer. The method has an advantage in that the column comprising silica carriers can be used repeatedly. However, the isolation of a nucleic acid from whole blood (which is a biological material) cannot be conducted because the column clogs. As a result, the method has disadvantages in that only a small amount of a nucleic acid can be collected. In addition, the apparatus utilized for the method is very expensive.

A method for separating a nucleic acid from a biological material (for example, whole blood, urine) utilizing silica particles as solid phase carrier is also known (JP-A-2-289596 and JP-B-7-13077).

However, in such a method that utilizes silica particles as a carrier, complicated procedures are required (for example, centrifugation must be conducted many times). In addition, after adding the sample solution to the particles, the method requires a mixing operation by vigorously stirring with a vortex mixer in order to sufficiently mix the sample solution and the particles. Because of the vigorous stirring, a nucleic acid contained in the sample tends to be degraded, and as a result, a long-chain nucleic acid can hardly be separated.

As described above, there are various problems related to non-specifically immobilizing a large amount of nucleic acids.

The nucleic acid-bondable magnetic carrier used in the present invention comprises magnetic silica particles containing a superparamagnetic metal oxide, wherein the magnetic silica particles have a specific surface of about 100 to about 800 m²/g.

In one embodiment of the present invention, the magnetic silica particles are a composite of a superparamagnetic metal oxide having a surface covered with silica and an inorganic porous matrix material composed of fine silica particles, and are substantially spherical.

In another embodiment of the present invention, the superparamagnetic metal oxide is iron oxide.

In still another embodiment of the present invention, the superparamagnetic metal oxide is contained in an amount of about 10 to about 60 percent by weight.

The magnetic silica particles used in the present invention have an average surface pore diameter of 0.1 to 60 nm, a pore volume of less than 0.5 ml/g, and a particle diameter of 0.5 to 15 µm.

According to one aspect of the invention, a method for isolating a nucleic acid includes the steps of:
mixing a nucleic acid-bondable magnetic carrier which comprises magnetic silica particles containing a superparamagnetic metal oxide; a material containing a nucleic acid and a solution for extracting the nucleic acid so as to form a sample solution;
separating the magnetic carrier to which the nucleic acid has been bonded from the sample solution using a magnetic field; and
eluting the nucleic acid from the magnetic carrier to which the nucleic acid has been bonded, wherein the magnetic silica particles have a pore volume of less than 0.5 ml/g, an average surface pore diameter of 0.1 to 60 nm, and a particle diameter of 0.5 to 15 µm.

In one embodiment of the present invention, the nucleic acid is a nucleic acid in a plasmid or an amplified product.

In another embodiment of the present invention, the solution for extracting the nucleic acid contains a chaotropic material.

In still another embodiment of the present invention, the chaotropic material is selected from guanidine salts, sodium iodide, potassium iodide, sodium thiocyanate, sodium isothiocyanate, urea and combinations thereof.

In still another embodiment of the present invention, the method utilizes an elution buffer in the eluting step.

In still another embodiment of the present invention, the buffer is TE buffer or sterilized water.

According to another aspect of the invention, a method for detecting a nucleic acid includes the steps of:
mixing a nucleic acid-bondable magnetic carrier which comprises magnetic silica particles containing a superparamagnetic metal oxide crystal; a material containing a nucleic acid and a solution for extracting the nucleic acid so as to form a sample solution;
separating the magnetic carrier to which the nucleic acid has been bonded from the sample solution using a magnetic field;
eluting the nucleic acid from the magnetic carrier; and
detecting a target nucleic acid, wherein the magnetic silica particles have a pore volume of less than 0.5 ml/g, an average surface pore diameter of 0.1 to 60 nm, and a particle diameter of 0.5 to 15 µm.

In one embodiment of the present invention, the method further includes the step of amplifying the eluted nucleic acid.

According to still another aspect of the invention, a kit for isolating a nucleic acid includes a nucleic acid-bondable magnetic carrier which comprises magnetic silica particles containing a superparamagnetic metal oxide; and a solution for extracting the nucleic acid, the magnetic silica particles have a pore volume of less than 0.5 ml/g, an average surface pore diameter of 0.1 to 60 nm, and a particle diameter of 0.5 to 15 µm.

Thus, the invention described herein makes possible the advantages of (1) using a magnetic carrier which is capable of non-specifically adsorbing a large amount of nucleic acids and therefore has an excellent collection efficiency; (2) providing a method for isolating a nucleic acid, which is capable of non-specifically adsorbing a large amount of nucleic acids and therefore has an excellent collection efficiency; (3) providing a method for isolating a nucleic acid having an excellent operability; (4) providing a method for isolating a nucleic acid, which can be easily automatized; (5) providing a method for detecting a nucleic acid having an excellent operability; (6) providing a method for detecting a nucleic acid, which can be easily automatized; and (7) providing a kit utilized for such method.

The nucleic acid-bondable magnetic carrier used in the present invention are magnetic silica particles comprising a superparamagnetic metal oxide. The magnetic silica particles of the present invention are capable of bonding a nucleic acid and separating solid and liquid by utilizing a magnetic field.

A nucleic acid is bonded to the magnetic silica particle of the present invention via a hydrogen bond which is formed between a hydroxyl group on the particle surface and a base of the nucleic acid.

The magnetic silica particles used in the present invention have a specific surface of about 100 to about 800 m²/g, preferably about 200 to about 600 m²/g, and more preferably about 300 to about 500 m²/g. The specific surface can be determined by a nitrogen gas adsorption method which is defined by JIS K1150 "Test methods for silica gels". In the case where the specific surface of the magnetic silica particles is less than about 100 m²/g, the capability of adsorbing a nucleic acid is insufficient. As a result, only a small amount of nucleic acids can be collected in many cases. In the case where the specific surface exceeds about 800 m²/g, a pore volume of the particle becomes too large. As a result, since only a small amount of sample solution can be collected by elution, only a small amount of nucleic acids can be collected in many cases.

In a preferred embodiment of the invention, the magnetic silica particles of the present invention are a composite of the superparamagnetic metal oxide having a surface covered with silica and an inorganic porous matrix material composed of fine silica particles. The magnetic silica particles are substantially spherical.

The superparamagnetic metal oxide used in the present invention refers to the metal oxide which is responsive to a magnetic field variation but is not permanently magnetized, and has a small residual magnetization.

A preferred example of the superparamagnetic metal oxide is iron oxide. As iron oxide, triiron tetraoxide (Fe₃O₄), iron sesquioxide (γFe₂O₃), which is obtained by gradually oxidizing triiron tetraoxide, and the like may be used. Triiron tetraoxide is especially preferably used. The superparamagnetic metal oxide is preferably in the form of particle, and more preferably in the form of substantially spherical particles. The diameter of the superparamagnetic metal oxide is preferably in the range of about 0.2 to about 0.4 µm, more preferably in the range of about 0.25 to about 0.30 µm. Since triiron tetraoxide having a substantially spherical form has an especially small residual magnetization and a smooth surface, it can be used repeatedly in separating operations. Furthermore, the magnetic silica particle containing triiron tetraoxide has excellent stability in neutral and weak acidic aqueous solutions and is capable of being stored more than two years in the solution.

The amount of the superparamagnetic metal oxide contained in the magnetic silica particles of the present invention may vary depending on the magnetization intensity of the metal oxide; however, the amount is preferably in the range of about 10 to about 60 percent by weight, more preferably in the range of about 20 to about 40 percent by weight. By providing the superparamagnetic metal oxide in the magnetic silica particles in such a preferable range, the magnetic carrier (i.e., the magnetic silica particle) can be rapidly separated from the sample solution utilizing commercially available magnets.

Furthermore, an average surface pore diameter of the magnetic silica particle is in the range of about 0.1 to about 60 nm, and preferably in the range of about 0.5 to about 10 nm. A pore volume of the magnetic silica particles is less than 0.5 ml/g, and preferably in the range of about 0.1 to about 0.5 ml/g. The surface pore diameter and the pore volume are determined by a nitrogen gas adsorption method which is defined according to JIS K1150 "Test methods for silica gels".

The specific surface and the pore volume depend on the size of surface pore diameter. The larger the surface pore diameter is, the larger the specific surface are and the pore volume are. The larger the specific surface is, the larger the adsorbed amount of nucleic acid is; however, the collected amount of nucleic acid tends to decrease because the pore volume also becomes large. In the above-mentioned range of the specific surface and the pore volume, a remarkably large amount of nucleic acid can be collected.

The particle diameter of the magnetic silica particle is in the range of about 0.5 to about 15 µm, and preferably in the range of about 1 to about 10 µm. In such a range, the magnetic silica particle has an excellent dispersibility from mixing.

The most preferred magnetic silica particles satisfy the following requirements: (1) they contain a superparamagnetic iron oxide; (2) they have a specific surface of about 100 to about 800 m²/g; (3)the iron oxide is almost covered with silica; (4) they are a composite of the iron oxide covered with silica and an inorganic porous matrix material composed of fine silica particles; (5) they contain the iron oxide in an amount of about 10 to about 60 percent by weight; (6) they have an average surface pore diameter of about 0.1 to about 60 nm; (7) they have a pore volume of less than 0.5 ml/g; and (8) they have a particle diameter of about 0.5 to about 15

The magnetic silica particles used in the present invention can be produced according to the method described, for example, in Japanese Patent Publication No. 6-47273. For example, triiron tetraoxide (Fe₃O₄)particles are added to a tetraethoxysilane/alcohol solution and the iron oxide particle is dispersed in the solution by ultrasonication. A hydrolytic catalyst for tetraethoxysilane is added to the dispersion and silica particles are deposited on the surface of the iron oxide particle while the iron oxide particle is dispersed by ultrasonication. Sodium silicate, an organic solvent (for example, toluene) and a surfactant (for example, sorbitan monostearate) are added to the dispersion thus obtained, so as to form W/O type emulsion. The emulsion is added to an aqueous ammonium sulfate solution and the mixture is thoroughly stirred. The emulsion is then filtered to separate the particles from the emulsion. The particles are washed with water, precipitated in alcohol and dried to obtain a desired spherical silica particle.

The magnetic silica particles used in the present invention have a specific surface much larger than that of a conventional magnetic particle. Therefore the magnetic silica particles used in the present invention are capable of non-specifically adsorbing a large amount of nucleic acids. Furthermore, since the magnetic silica particles have excellent dispersibility, they are easily mixed with a sample containing a nucleic acid and a solution for extracting the nucleic acid. As a result, a large amount of nucleic acids can be easily collected by elution.

A method for isolating a nucleic acid of the present invention includes the steps of mixing a nucleic acid-bondable magnetic carrier, which is a magnetic silica particle containing a superparamagnetic metal oxide; a material containing a nucleic acid and a solution for extracting the nucleic acid so as to form a sample solution; separating the magnetic carrier to which the nucleic acid has been bonded from the sample solution using a magnetic field; and eluting the nucleic acid from the magnetic carrier to which the nucleic acid has been bonded, wherein the magnetic silica particles have a pore volume of less than 0.5 ml/g, an average surface pore diameter of 0.1 to 60 nm, and a particle diameter of 0.5 to 15 µm.

The step of mixing the nucleic acid-bondable magnetic carrier, the material containing the nucleic acid and the solution for extracting the nucleic acid can be conducted using, for example, a commercially available vortex mixer. The mixing step can also be conducted by shaking or inverting a sample tube containing the above-mentioned contents.

The step of separating the magnetic carrier to which the nucleic acid has been bonded from the sample solution by using a magnetic field can be conducted utilizing a magnet. The magnet having a magnetic flux density of preferably about 200 to about 4000 Gauss, and more preferably about 2000 Gauss can be used. For example, the magnet is put close to the side wall of the sample tube containing the nucleic acid-bondable magnetic carrier, the material containing the nucleic acid and the solution for extracting the nucleic acid so as to bring the magnetic carrier together at the side wall of the tube. The magnetic carrier is then separated from the solution.

The step of eluting the nucleic acid from the magnetic carrier to which the nucleic acid has been bonded can be conducted, for example, as follows. The magnetic carrier to which the nucleic acid has been bonded is washed several times with an aqueous solution of about 70% ethanol and then dried. Thereafter, a solution having a low ionic strength (for example, Tris-EDTA buffer (TE buffer), sterilized water) is added to the carrier. In such a manner, the nucleic acid which is bonded to the magnetic carrier can be eluted.

The method for isolating a nucleic acid of the present invention does not require specifically bonding a single strand DNA or RNA to the magnetic carrier.

The material containing the nucleic acid used in the present invention is a biological material containing protein, membrane, DNA or RNA, low molecular weight nucleic acid and the like. Examples of the biological material include a bacteriophage, virus and bacteria containing protein, membrane, DNA or RNA, low molecular weight nucleic acid and the like, and combinations thereof. For the purpose of purification, the nucleic acid may also be a nucleic acid in plasmid or amplified product.

The solution for extracting the nucleic acid used in the present invention includes buffer containing e.g. chaotropic material, EDTA or tris HCl.
Examples of chaotropic material include guanidine salts, sodium iodide, potassium iodide, sodium thiocyanate, sodium isothiocyanate and urea. The chaotropic material can be used alone or in combination. The concentration of chaotropic material in the solution is preferably in the range of about 1 to about 10 mole/l, and more preferably in the range of about 3 to about 5 mole/l.

Preferred examples of the solution for extracting the nucleic acid in the present invention include guanidine thiocyanate, Triton X-100, and Tris-HCl buffer.

An elution buffer such as TE buffer or sterilized water can be used in order to elute and collect the nucleic acid.

In the method for isolating a nucleic acid of the present invention utilizing magnetic silica particles as a solid phase carrier, the specific surface of the solid phase carrier is several times as large as that in the case where conventional porous glass or magnetic fine particle is used. As a result, according to the method of the present invention, a large amount of nucleic acids can be collected.

According to the method for isolating a nucleic acid of the present invention, the extracted nucleic acid can be bonded to the carrier in a high concentration salt solution. Furthermore, the elution of the nucleic acid can be conducted in a solution having a low ionic strength (for example, TE buffer, sterilized water).

A preferred example of the method for isolating a nucleic acid of the present invention includes the following procedures.
(1) A solution for extracting a nucleic acid is put into a microcentrifuge tube. Then, a whole blood sample is added to and mixed with the solution.
(2) A dispersion containing a magnetic silica particle in sterilized water is added into the tube.
(3) The sample in the tube is repeatedly mixed and allowed to settle at an appropriate interval.
(4) The above-mentioned tube is set up at a magnetic stand which conforms to the shape of the tube so as to bring the magnetic silica particle together at the side wall of the tube.
(5) The solution is drawn off by suction with a filter tip. The solution may also be drawn off by decantation (i.e., inverting the magnetic stand with the tube being set up); however, this operation has contamination problems due to splashing of waste liquid. Therefore, the filter tip is preferably used for drawing off the solution.
(6) After taking out the tube from the magnetic stand, a washing buffer containing guanidine thiocyanate is added into the tube.
(7) After sufficiently mixing the magnetic silica particles and the washing buffer, the tube is set up at a magnetic stand. Then, the solution is drawn off in the above-mentioned manner.
(8) The washing procedure described in (6) and (7) is performed again.
(9) The magnetic silica particles are washed with appropriate organic solvent(s) (for example, about 70% ethanol solution and acetone solution) in the above-mentioned manner so as to remove high concentration guanidine thiocyanate.
(10) Again, the magnetic silica particles are washed with appropriate organic solvent(s) (for example, about 70% ethanol solution and acetone solution).
(11) The tube is placed and left in a heat block at an appropriate temperature (for example, an elevated temperature of about 56°C) so as to substantially remove the organic solvent by evaporation.
(12) Sterilized water is added into the tube. The tube is then placed in the heat block at an appropriate temperature (for example, an elevated temperature of about 56°C). Thereafter, the procedure described in (3) is repeated.
(13) The tube is set up at the magnetic stand. Then, the solution to be collected is moved to another tube with the filter tip.
(14) If desired, the collected solution may be stored at an appropriate temperature (for example, about -70°C).

A nucleic acid isolated by the method of the present invention may be amplified by a method of amplifying a nucleic acid, and may be detected by a detectable probe, if necessary.

Examples of the method of amplifying a nucleic acid include Polymerase Chain Reaction (PCR) method and Nucleic Acid Sequence Based Amplification (NASBA) method. A preferred example of the method of amplifying a nucleic acid includes the steps of: (A) denaturing a target nucleic acid to obtain a single strand nucleic acid, if necessary; (B) reacting the single strand nucleic acid with forward and reverse primers having a nucleotide sequence complementary to that of the target nucleic acid and four kinds of dNTP in a buffer containing thermostable DNA polymerase, so as to anneal the primers to the single strand nucleic acid and initiate a primer extension; (C) separating the extended product to obtain a single strand; and (D) repeating the steps (B) and (C).

According to the present invention, if necessary, the target nucleic acid is detected, for example, by hybridizing a labeled probe with the amplified product of the above-mentioned amplifying method.

As a labeled probe, an oligonucleotide having a nucleotide sequence complementary to that of the target nucleic acid, which is capable of binding a label material or label-binding material thereto, can be used.

Examples of the label material include enzymes such as alkaline phosphatase, peroxidase, galactosidase, fluorescent materials, and radioactive materials. Examples of the label-binding material include biotin and digoxigenin. The label material can be bound to the probe via biotin, digoxigenin or avidin.

A method for incorporating the label material into the probe includes a method for synthesizing a probe utilizing dNTP which is capable of binding a label material or label-binding material thereto.

As a method for detecting a nucleic acid to which a labeled probe is bound, any known methods, such as Northern hybridization and Southern hybridization, can be used.

In detecting the label, for example, in the case where alkaline phosphatase is used as the label material, only a nucleic acid hybridized with the labeled probe is luminesced when the label material is reacted with a chemiluminescent substrata (for example, 1,2-dioxetane compound (PPD)). The size and position on electrophoresis of a target nucleic acid can be determined by exposing the luminesced nucleic acid on an X-ray film.

A kit for isolating a nucleic acid of the present invention comprises a nucleic acid-bondable magnetic carrier which comprises magnetic silica particles containing a superparamagnetic metal oxide and a solution for extracting the nucleic acid, wherein the magnetic silica particles have a pore volume of less than 0.5 ml/g, an average surface pore diameter of 0.1 to 60 nm, and a particle diameter of 0.5 to 15 µm.

The magnetic carrier and the solution for extracting the nucleic acid contained in the kit are as described above.

According to the present invention, since the magnetic silica particles have a specific surface much larger than that of conventional magnetic particles, the magnetic silica particles are capable of non-specifically adsorbing a large amount of nucleic acids. Furthermore, since the magnetic silica particles have excellent dispersibility, it is easily mixed with a sample containing a nucleic acid and a solution for extracting the nucleic acid. As a result, a large amount of nucleic acids can be collected by elution. Accordingly, by utilizing the magnetic silica particles, a method for isolating and detecting a nucleic acid, and a kit for isolating a nucleic acid, having a high nucleic acid yield, are provided.

Hereinafter, the present invention will be described by way of an illustrative example.

### Examples

The magnetic silica particles used in the examples of the present invention are described as follows: (i)the particle diameter is 1 to 10 µm; (ii) the content of triiron tetraoxide is 30 percent by weight; (iii) the specific surface is 400 m²/g; (iv) the pore volume is 0.15 ml/g; and (v) the average surface pore diameter is about 1.20 nm. The particles are manufactured by Suzuki Yushi Co. Ltd.

The particle diameter, specific surface, pore volume, and surface pore diameter of the magnetic silica particles are determined by a method which is defined by JIS K1150 "Test methods for silica gels"

Since the magnetic silica particles are not easily dispersed in an aqueous solution, it is inconvenient in operation if the particles are directly dispersed in the sample solution. Therefore, the dispersion containing 0.5 g of the particles in 1 cc of sterilized water was previously prepared.

### Example 1 : A method for isolating a nucleic acid from a biological material

A whole blood sample positive to methicillin resistant Staphylococcus aureus (MRSA) was used as a biological material. Tris-HCl buffer containing 5M guanidine thiocyanate and Triton X-100 was used as a solution for extracting a nucleic acid. Tris-HCl buffer containing guanidine thiocyanate was also used as a washing buffer. 70% ethanol solution and acetone solution were used for removing high concentration salt. Furthermore, sterilized water was used as an eluent for collecting the nucleic acid which is bonded to the solid phase carrier (magnetic silica particle).

The procedures of the Example are as follows.
(1) 900 µl of a solution for extracting a nucleic acid was put into 1.5 cc microcentrifuge tube. Then, 100 µl of whole blood sample was added to and mixed with the solution.
(2) 140 µl of the above-mentioned dispersion containing magnetic silica particles in sterilized water was added into the tube.
(3) The sample in the tube was mixed and allowed to settle for two minutes, and the same procedure was performed four more times.
(4) The above-mentioned tube was set up at a magnetic stand which conforms to the shape of the tube so as to bring the magnetic silica particles together at the side wall of the tube.
(5) The solution was drawn off by suction with a filter tip.
(6) After taking out the tube from the magnetic stand, 1 cc of washing buffer containing guanidine thiocyanate was added into the tube.
(7) After thoroughly mixing the magnetic silica particle and the washing buffer, the tube was set up at the magnetic stand. Then, the solution was drawn off in the above-mentioned manner.
(8) The washing procedure was performed again.
(9) The magnetic silica particle was washed with 1 cc of 70% ethanol solution in the above-mentioned manner so as to remove the high concentration of guanidine thiocyanate.
(10) Again, the magnetic silica particles were washed with 1 cc of 70% ethanol solution and 1 cc of acetone solution.
(11) The tube was placed in a heat block at about 56°C and left for ten minutes so as to substantially remove acetone from the tube and the magnetic silica particle by evaporation.
(12) 100 µl of sterilized water was added into the tube. The tube was then placed in the heat block at about 56°C. Thereafter, the sample in the tube was mixed and allowed to settle for two minutes, and the same procedure was performed four more times.
(13) The tube was set up at the magnetic stand. Then, the solution to be collected was moved to another tube with the filter tip. The volume of the collected solution is usually about 70 µl.
(14) In the case where the collected solution is stored, it was stored at -70°C.

The concentration of nucleic acid in the solution thus collected was determined by measuring absorbance (OD, at 260 nm) by absorption photometry. The amount of collected nucleic acid was determined with multiplying the concentration by the volume of the collected solution.

### Comparative Example 1

For comparison, a commercially available kit for extracting a nucleic acid (Isoquick, produced by Microprobe Inc.) was used. The extraction of the nucleic acid was conducted in the following manner. First, the biological sample of Example 1 was reacted with a chaotropic material, resulting in disruption of cell membrane and inhibition of nuclease activity. Then, the nucleic acid was moved into the aqueous phase while the remaining materials were left in organic phase. Finally, the nucleic acid was precipitated with alcohol so as to be taken out from aqueous phase.

The concentration and collected amount of nucleic acid thus collected were determined in the above-mentioned manner.

The results of Example 1 and Comparative Example 1 are shown in Table 1 below.

**Table 1 : An amount of collected nucleic acid**

| | Example 1 | Comparative Example 1 |
|---|---|---|
| The collected amount (ng) | 108.0 | 74.5 |

As is apparent from Table 1, the amount of collected nucleic acid in Example 1 (according to the present invention) is higher than that in Comparative Example 1.

### Example 2 : The test for determining a collection efficiency using a nucleic acid sample having a known MRSA concentration

After methicillin resistant Staphylococcus aureus (MRSA) was cultured, the concentration (the number of MRSA) was calculated. The nucleic acid which had been extracted from MRSA by acid guanidium thiocyanate phenolchloroform (AGPC) method was added to whole blood from healthy volunteers to prepare a test sample. The nucleic acid solution without whole blood was used as a control sample. Using both samples, the collection efficiency of the nucleic acid using the magnetic silica particle of Example 1 was determined.

### Test sample

100 µl of the nucleic acid solutions from MRSA of 10⁴ cells/100 µl and 10⁵ cells/100 µl were respectively added to whole blood from healthy volunteers to prepare the test samples.

### A method for isolating a nucleic acid

The nucleic acid was extracted in the same manner as in Example 1.

### A method for amplifying a nucleic acid

PCR method was conducted using two optimum primers (SEQUENCE ID NOS. 1 and 2) from mecA gene sequences. 30 cycles of one minute at 94°C, one minute at 55°C, and one minute at 75°C were conducted.

### A method for detecting the nucleic acid

A dot-plot method described below was employed as a method for detecting the nucleic acid. A probe (SEQUENCE ID NO. 3) was produced from mecA gene sequences. The probe was labeled with alkaline phosphatase. Sandwich hybridization was conducted for the amplified test sample by using the labeled probe. After addition of 1,2-dioxetane compound (PPD) as a luminescent substrate, the luminescence level for the sample was measured using a detection meter.

### Collection efficiency

The amount of collected nucleic acid was calculated based on the measured luminescence level by a well-known calculation method. The collection efficiency was determined with dividing the collected amount of the test sample by that of the control sample.

### Comparative Example 2

The nucleic acid was extracted in the same manner as in Comparative Example 1 using a commercially available kit for extracting a nucleic acid (Isoquick, produced by Microprobe Inc.). The collection efficiency was calculated in the same manner as in Example 2.

The results of Example 2 and Comparative Example 2 are shown in Table 2 below.

**Table 2: Collection efficiency**

| Sample | Example 2 | Comparative Example 2 |
|---|---|---|
| 10⁴ cells/100 µl | 50% | 12% |
| 10⁵ cells/100 µl | 30% | 10% |

As is apparent from Table 2, the collection efficiency of the nucleic acid in Example 2 (according to the present invention) is higher than that in Comparative Example 2.

### Example 3 : The test for determining a collection efficiency using a linear DNA fragment

A whole blood sample of a healthy volunteer was used as a biological material. A linear DNA fragment described below was added to the sample and recovered therefrom. The nucleic acid was recovered from the biological material according to the isolation method in Example 1.

### A linear DNA fragment

10 ng/µl of pBluescriptII/Scal fragment (2.96 kbp) or 40 ng/µl of y/HindIII digest was used.

### Detection

The collected nucleic acid was detected by the dot-plot method in the same manner as in Example 2. The collection efficiency of the nucleic acid was calculated in the same manner as in Example 2.

### Comparative Example 3

For comparison, the nucleic acid was isolated in the following procedure. After the sample of Example 3 was treated with a prescribed extracting solution, the nucleic acid was adsorbed with silica resin. The silica resin was collected in a filter cup and washed. Finally, the nucleic acid was eluted with sterilized water or diluted TE buffer. The eluted nucleic acid was collected using a commercially available kit for extracting a nucleic acid (ClearCut Miniprep Kit, produced by Stratagene Inc.). The procedure was as follows; (a) 100 µl of the sample, 100 µl of Solution 3 (which is contained' in the above-mentioned kit), and 10 µl of the silica dispersion were put into the tube of the kit; (b) After mixing, the particles were collected with a spin column and a supernatant was removed; (c) The particles were washed twice with 500 µl of washing buffer; and (d) 100 µl of TE buffer was added to the washed particles and a supernatant was collected.

The collection efficiency was calculated in the same manner as in Example 2.

The results of Example 3 and Comparative Example 3 are shown in Tables 3 and 4 below.

**Table 3: 10 ng/µl of pBluescriptII/ScaI sample**

| | Example 3 | Comparative Example 3 |
|---|---|---|
| Collection efficiency | 50% | 40% |

**Table 4: 40 ng/µl of γ/HindIII sample**

| | Example 3 | Comparative Example 3 |
|---|---|---|
| Collection efficiency | 30% | 20% |

As is apparent from Tables 3 and 4, the collection efficiency of the. DNA fragment in Example 3 (according to the present invention) is higher than that in Comparative Example 3.

The method for isolating the nucleic acid using the magnetic silica particles Of the present invention is capable of non-specifically adsorbing a large amount of nucleic acids and therefore has an excellent collection efficiency. Furthermore, since the method of the present invention has excellent operability, it is useful for research or the pre-treatment of clinical specimen wherein a large number of specimens must be treated in a short time. Since the method of the present invention is also capable of effectively extracting DNA and/or RNA, it is useful for a pre-treatment of various methods of amplifying a nucleic acid. In addition, since the method of the present invention separates the magnetic particle from the sample solution using a magnetic field, it can be easily automatized.

Various other modifications will be apparent to and can be readily made by those skilled in the art without departing from the scope of this invention. Accordingly, it is not intended that the scope of the claims appended hereto be limited to the description as set forth herein, but rather that the claims be broadly construed.

## Claims

1. A method for isolating a nucleic acid, comprising the steps of:
mixing a nucleic acid-bondable magnetic carrier which is magnetic silica particles comprising a superparamagnetic metal oxide;
a material containing a nucleic acid and a solution for extracting the nucleic acid so as to form a sample solution;
separating the magnetic carrier to which the nucleic acid has been bonded from the sample solution using a magnetic field; and
eluting the nucleic acid from the magnetic carrier to which the nucleic acid has been bonded,
wherein the magnetic silica particles have a pore volume of less than 0.5 ml/g, an average surface pore diameter of 0.1 to 60 nm, and a particle diameter of 0.5 to 15 µm.

2. A method according to claim 1, wherein the magnetic silica particles are spherical.

3. A method according to claim 1, wherein the superparamagnetic metal oxide is iron oxide.

4. A method according to claim 1, wherein the superparamagnetic metal oxide is contained in an amount of 10 to 60 percent by weight.

5. A method for amplifying a nucleic acid, comprising the steps of:
mixing a nucleic acid-bondable magnetic carrier which is magnetic silica particles comprising a superparamagnetic metal oxide;
a material containing a nucleic acid and a solution for extracting the nucleic acid so as to form a sample solution;
separating the magnetic carrier to which the nucleic acid has been bonded from the sample solution using a magnetic field;
eluting the nucleic acid from the magnetic carrier to which the nucleic acid has been bonded; and
amplifying the eluted nucleic acid,
wherein the magnetic silica particles have a pore volume of less than 0.5 ml/g, an average surface pore diameter of 0.1 to 60 nm, and a particle diameter of 0.5 to 15 µm.

6. A method for detecting a nucleic acid comprising the steps of:
mixing a nucleic acid-bondable magnetic carrier which comprises magnetic silica particles containing a superparamagnetic metal oxide crystal;
a material comprising a nucleic acid and a solutions for extracting the nucleic acid so as to form a sample solution;
separating the magnetic carder to which the nucleic acid has been bonded from the sample solution using a magnetic field;
eluting the nucleic acid from the magnetic carrier; and
detecting a target nucleic acid,
wherein the magnetic silica particles have a pore volume of less than 0.5 ml/g, an average surface pore diameter of 0.1 to 60 nm, and a particle diameter of 0.5 to 15 µm.3>.

7. A method for detecting a nucleic acid according to claim 6, further comprising the step of amplifying the eluted nucleic acid.

8. Use of a magnetic carrier comprising magnetic silica particles comprising a superparamagnetic metal oxide, for the isolation of a nucleic acid,
wherein the magnetic silica particles have a pore volume of less than 0.5 ml/g, an average surface pore diameter of 0.1 to 60 nm, and a particle diameter of 0.5 to 15 µm.

9. Use of a magnetic carrier comprising magnetic silica particles comprising a superparamagnetic metal oxide, for the amplification of a nucleic acid,
wherein the magnetic silica particles have a pore volume of less than 0.5 ml/g, an average surface pore diameter of 0.1 to 60 nm, and a particle diameter of 0.5 to 15 µm.

10. Use of a magnetic carrier comprising magnetic silica particles comprising a superparamagnetic metal oxide, for the detection of a nucleic acid,
wherein the magnetic silica particles have a pore volume of less than 0.5 ml/g, an average surface pore diameter of 0.1 to 60 nm, and a particle diameter of 0.5 to 15 µm.

11. A kit for isolating a nucleic acid, comprising a nucleic acid-bondable magnetic carrier which comprises magnetic silica particles comprising a superparamagnetic metal oxide, and a solution for extracting the nucleic acid,
wherein the magnetic silica particles have a pore volume of less than 0.5 ml/g, an average surface pore diameter of 0.1 to 60 nm, and a particle diameter of 0.5 to 15 µm.

12. A kit for detecting a nucleic acid, comprising a nucleic acid-bondable magnetic carrier which comprises magnetic silica particles comprising a superparamagnetic metal oxide, and a solution for extracting the nucleic acid,
wherein the magnetic silica particles have a pore volume of less than 0.5 ml/g, an average surface pore diameter of 0.1 to 60 nm, and a particle diameter of 0.5 to 15 µm.

13. The method of any one of claims 1, 5 and 6, the use of any one of claims 8, 9 and 10, and/or the kit of any one of claims 11 and 12, wherein the magnetic silica particles have a pore volume of 0.1 to less than 0.5 ml/g.

## Patentansprüche

1. Verfahren zum Isolieren einer Nucleinsäure, umfassend die Schritte:
Mischen eines eine Nucleinsäure bindenden magnetischen Trägers, bei dem es sich um magnetische Siliciumdioxidteilchen handelt, die ein superparamagnetisches Metalloxid umfassen;
eines eine Nucleinsäure enthaltenden Materials und einer Lösung zum Extrahieren der Nucleinsäure, um so eine Probenlösung zu bilden;
Abtrennen des magnetischen Trägers, an den die Nucleinsäure gebunden wurde, aus der Probenlösung unter Verwendung eines magnetischen Felds; und
Eluieren der Nucleinsäure vom magnetischen Träger, an den die Nucleinsäure gebunden wurde,
wobei die magnetischen Siliciumdioxidteilchen ein Porenvolumen von weniger als 0,5 ml/g, einen mittleren Porendurchmesser der Oberfläche von 0,1 bis 60 nm und einen Teilchendurchmesser von 0,5 bis 15 *µ*m aufweisen.

2. Verfahren nach Anspruch 1, wobei die magnetischen Siliciumdioxidteilchen kugelförmig sind.

3. Verfahren nach Anspruch 1, wobei das superparamagnetische Metalloxid Eisenoxid ist.

4. Verfahren nach Anspruch 1, wobei das superparamagnetische Metalloxid in einer Menge von 10 bis 60 Gew.-% enthalten ist.

5. Verfahren zur Amplifikation einer Nucleinsäure, umfassend die Schritte:
Mischen eines eine Nucleinsäure bindenden magnetischen Trägers, bei dem es sich um magnetische Siliciumdioxidteilchen handelt, die ein superparamagnetisches Metalloxid umfassen;
eines eine Nucleinsäure enthaltenden Materials und einer Lösung zum Extrahieren der Nucleinsäure, um so eine Probenlösung zu bilden;
Abtrennen des magnetischen Trägers, an den die Nucleinsäure gebunden wurde, aus der Probenlösung unter Verwendung eines magnetischen Felds; und
Eluieren der Nucleinsäure vom magnetischen Träger, an den die Nucleinsäure gebunden wurde, und
Amplifikation der eluierten Nucleinsäure;
wobei die magnetischen Siliciumdioxidteilchen ein Porenvolumen von weniger als 0,5 ml/g, einen mittleren Porendurchmesser der Oberfläche von 0,1 bis 60 nm und einen Teilchendurchmesser von 0,5 bis 15 *µ*m aufweisen.

6. Verfahren zum Nachweis einer Nucleinsäure, umfassend die Schritte:
Mischen eines eine Nucleinsäure bindenden magnetischen Trägers, welcher magnetische Siliciumdioxidteilchen umfasst, die einen superparamagnetischen Metalloxidkristall enthalten;
eines eine Nucleinsäure umfassenden Materials und einer Lösung zum Extrahieren der Nucleinsäure, um so eine Probenlösung zu bilden;
Abtrennen des magnetischen Trägers, an den die Nucleinsäure gebunden wurde, aus der Probenlösung unter Verwendung eines magnetischen Felds;
Eluieren der Nucleinsäure vom magnetischen Träger, und
Nachweis einer Ziel-Nucleinsäure,
wobei die magnetischen Siliciumdioxidteilchen ein Porenvolumen von weniger als 0,5 ml/g, einen mittleren Porendurchmesser der Oberfläche von 0,1 bis 60 nm und einen Teilchendurchmesser von 0,5 bis 15 µm aufweisen.

7. Verfahren zum Nachweis einer Nucleinsäure nach Anspruch 6, das weiter den Schritt der Amplifikation der eluierten Nucleinsäure umfasst.

8. Verwendung eines magnetischen Trägers, der magnetische Siliciumdioxidteilchen umfasst, welche ein superparamagnetisches Metalloxid umfassen, zum Isolieren einer Nucleinsäure,
wobei die magnetischen Siliciumdioxidteilchen ein Porenvolumen von weniger als 0,5 ml/g, einen mittleren Porendurchmesser der Oberfläche von 0,1 bis 60 nm und einen Teilchendurchmesser von 0,5 bis 15 µm aufweisen.

9. Verwendung eines magnetischen Trägers, der magnetische Siliciumdioxidteilchen umfasst, welche ein superparamagnetisches Metalloxid umfassen, zur Amplifikation einer Nucleinsäure,
wobei die magnetischen Siliciumdioxidteilchen ein Porenvolumen von weniger als 0,5 ml/g, einen mittleren Porendurchmesser der Oberfläche von 0,1 bis 60 nm und einen Teilchendurchmesser von 0,5 bis 15 µm aufweisen.

10. Verwendung eines magnetischen Trägers, der magnetische Siliciumdioxidteilchen umfasst, welche ein superparamagnetisches Metalloxid umfassen, zum Nachweis einer Nucleinsäure,
wobei die magnetischen Siliciumdioxidteilchen ein Porenvolumen von weniger als 0,5 ml/g, einen mittleren Porendurchmesser der Oberfläche von 0,1 bis 60 nm und einen Teilchendurchmesser von 0,5 bis 15 µm aufweisen.

11. Kit zum Isolieren einer Nucleinsäure, umfassend einen eine Nucleinsäure bindenden magnetischen Träger, der magnetische Siliciumdioxidteilchen umfasst, welche ein superparamagnetisches Metalloxid umfassen, und eine Lösung zur Extraktion der Nucleinsäure,
wobei die magnetischen Siliciumdioxidteilchen ein Porenvolumen von weniger als 0,5 ml/g, einen mittleren Porendurchmesser der Oberfläche von 0,1 bis 60 nm und einen Teilchendurchmesser von 0,5 bis 15 µm aufweisen.

12. Kit zum Nachweis einer Nucleinsäure, umfassend einen eine Nucleinsäure bindenden magnetischen Träger, der magnetische Siliciumdioxidteilchen umfasst, welche ein superparamagnetisches Metalloxid umfassen, und eine Lösung zur Extraktion der Nucleinsäure,
wobei die magnetischen Siliciumdioxidteilchen ein Porenvolumen von weniger als 0,5 ml/g, einen mittleren Porendurchmesser der Oberfläche von 0,1 bis 60 nm und einen Teilchendurchmesser von 0,5 bis 15 *µ*m aufweisen.

13. Verfahren nach einem der Ansprüche 1, 5 und 6, Verwendung nach einem der Ansprüche 8, 9 und 10 und/oder Kit nach einem der Ansprüche 11 und 12, wobei die magnetischen Siliciumdioxidteilchen ein Porenvolumen von 0,1 bis weniger als 0,5 ml/g aufweisen.

## Revendications

1. Procédé pour isoler un acide nucléique, comprenant les étapes consistant :
à mélanger un support magnétique capable de se lier à un acide nucléique,
ledit support étant des particules de silice comprenant un oxyde métallique
superparamagnétique ; un matériau contenant un acide nucléique et une solution pour extraire l'acide nucléique de façon à former une solution échantillon ;
à séparer le support magnétique auquel l'acide nucléique a été lié d'avec la solution échantillon en utilisant un champ magnétique ; et
à éluer l'acide nucléique d'avec le support magnétique auquel l'acide nucléique a été lié,
dans lequel les particules de silice magnétiques possèdent un volume de pore inférieur à 0,5 ml/g, un diamètre moyen de pore de surface de 0,1 à 60 nm, et un diamètre de particule de 0,5 à 15 *µ*m.

2. Procédé selon la revendication 1, dans lequel les particules de silice magnétiques sont sphériques.

3. Procédé selon la revendication 1, dans lequel l'oxyde métallique superparamagnétique est l'oxyde de fer.

4. Procédé selon la revendication 1, dans lequel l'oxyde métallique superparamagnétique est présent en une quantité de 10 à 60 pourcent en poids.

5. Procédé d'amplification d'un acide nucléique, comprenant les étapes consistant :
à mélanger un support magnétique capable de se lier à un acide nucléique, ledit support étant des particules de silice magnétiques comprenant un oxyde métallique superparamagnétique ; un matériau contenant un acide nucléique et une solution pour extraire l'acide nucléique de façon à former une solution échantillon ;
à séparer le support magnétique auquel l'acide nucléique a été lié d'avec la solution échantillon en utilisant un champ magnétique; et
à éluer l'acide nucléique d'avec le support magnétique auquel l'acide nucléique a été lié ; et
à amplifier l'acide nucléique élué,
dans lequel les particules de silice magnétiques possèdent un volume de pore inférieur à 0,5 ml/g, un diamètre moyen de pore de surface de 0,1 à 60 nm, et un diamètre de particule de 0,5 à 15 µm.

6. Procédé pour détecter un acide nucléique, comprenant les étapes consistant :
à mélanger un support magnétique capable de se lier à un acide nucléique
qui comprend des particules de silice magnétiques contenant un cristal
d'oxyde métallique superparamagnétique ; un matériau comprenant un acide
nucléique et une solution pour extraire l'acide nucléique de façon à former
une solution échantillon ;
à séparer le support magnétique auquel l'acide nucléique a été lié d'avec la
solution échantillon en utilisant un champ magnétique ;
à éluer l'acide nucléique d'avec le support magnétique ; et à détecter un acide nucléique cible,
dans lequel les particules de silice magnétiques possèdent un volume de pore inférieur à 0,5 ml/g, un diamètre moyen de pore surface de 0,1 à 60 nm, et un diamètre de particule de 0,5 à 15 *µ*m.

7. Procédé pour détecter un acide nucléique selon la revendication 6, comprenant de plus l'étape consistant à amplifier l'acide nucléique élué.

8. Utilisation d'un support magnétique comprenant des particules de silice magnétiques, comprenant un oxyde métallique superparamagnétique, pour isoler un acide nucléique dans lequel les particules de silice magnétiques possèdent un volume de pore inférieur à 0,5 ml/g, un diamètre moyen de pore de surface de 0,1 à 60 nm, et un diamètre de particule de 0,5 à 15 *µ*m.

9. Utilisation d'un support magnétique comprenant des particules de silice magnétiques, comprenant un oxyde métallique superparamagnétique, pour l'amplification d'un acide nucléique dans lequel les particules de silice magnétiques possèdent un volume de pore inférieur à 0,5 ml/g, un diamètre moyen de pore de surface de 0,1 à 60 nm, et un diamètre de particule de 0,5 à 15*µ*m.

10. Utilisation d'un support magnétique comprenant des particules de silice magnétiques, comprenant un oxyde métallique superparamagnétique, pour la détection d'un acide nucléique, dans lequel les particules de silice magnétiques possèdent un volume de pore inférieur à 0,5 ml/g, un diamètre moyen de pore de surface de 0,1 à 60 nm, et un diamètre de particule de 0,5 à 15 *µ*m.

11. Trousse pour isoler un acide nucléique, comprenant un support magnétique capable de se lier à un acide nucléique qui comprend des particules de silice magnétiques comprenant un oxyde métallique superparamagnétique, et une solution pour extraire l'acide nucléique, dans lequel les particules de silice magnétiques possèdent un volume de pore inférieur à 0,5 ml/g, un diamètre moyen de pore de surface de 0,1 à 60 nm, et un diamètre de particule de 0,5 à 15 *µ*m.

12. Trousse pour détecter un acide nucléique, comprenant un support magnétique capable de se lier à un acide nucléique qui comprend des particules de silice magnétiques comprenant un oxyde métallique superparamagnétique, et une solution pour extraire l'acide nucléique, dans lequel les particules de silice magnétiques possèdent un volume de pore inférieur à 0,5 ml/g, un diamètre moyen de pore de surface de 0,1 à 60 nm, et un diamètre de particule de 0,5 à 15 *µ*m.

13. Procédé selon l'une quelconque des revendications 1, 5 et 6, utilisation selon l'une quelconque des revendications 8, 9 et 10, et/ou trousse selon l'une quelconque des revendications 11 et 12, où les particules de silice magnétiques ont un volume de pore de 0,1 à moins de 0,5 ml/g.
